# EUROPEAN PATENT APPLICATION

(11) **EP 0 951 902 A1**
(43) Date of publication of application: **27.10.1999**
(21) Application number: 98302474.6
(22) Date of filing: 31.03.1998
(51) Int. Cl.: A61K 9/00, A23K 1/18, B65D 25/08, A01K 5/00

(54) **A feed mixture**

(71) Applicant: Campion, Edmund J.H., Winchmore Hill, London N21 3DA (GB)
(72) Inventor: Campion, Edmund J.H., Winchmore Hill, London N21 3DA (GB)
(74) Representative: Brown, John David

(57) **Abstract**

A feed mixture comprising a sealed package containing an animal foodstuff and a predetermined dose of a medicament; and a method of dosing an animal with a medicament comprising the steps of: providing a sealed package within which an animal foodstuff and a predetermined dose of the medicament are contained; opening the package; and feeding the animal with the feed mixture, the unitary nature of the predetermined dose of the medicament within each package allowing dosages to be varied by feeding a given animal packages in one or more sizes which can be administered singly, in multiples or in combinations thereof in order to achieve the correct therapeutic dose for the given animal.

## Description

THIS INVENTION RELATES TO a feed mixture and more particularly to a feed mixture of a foodstuff and a medicament.

There is a widely recognised need to eliminate parasite infestation in pets and domestic animals. Many doctors hold that parasites harboured by pets give rise to a constant undercurrent of human malaise; the vegetative forms of these parasites can and do find permanent lodging in the organs of the human body where they may produce effects from muscular pain to epilepsy; young children particularly are at risk from an intestinal parasite of dogs, occasionally with fatal results. Vets recommend dosing pets two to four times a year with worming medicines; other medicines such as those administered systemically for the control of fleas need to be given more frequently - up to once a month.

The dosing of pets is a domestic hurdle. Medicines in the right dose must be laid in; their diversity and the long intervals between doses make it difficult to remember what doses are required and when they should be administered. One method is to prepare a foodstuff and then mix in a medicament. Alternatively, the medicament, usually in the form of a pill, is laboriously crushed with inadequate tools to general wastage and mess; or pills are administered directly to the animal, with the risk of rejection of the pill, personal injury, or even of asphyxiating the animal. The whole process is problematic and has a nuisance value that discourages regular dosing. There are presently no solutions available for solving these problems. A system for easier dosing would not only be desirable; it would make a palpable contribution to public hygiene.

It is an object of the present invention to provide a feed mixture which ameliorates the above problems and allows the systemic medication of cats, dogs, and other animals, domestic and non-domestic, for the cure and prophylaxis of, for example, parasite infestation in a way convenient for the consumer. The ease of obtaining and administering the required medicinal dose would make a valuable contribution to public hygiene.

Accordingly, one aspect of the present invention provides a feed mixture comprising a sealed package containing an animal foodstuff and a predetermined dose of a medicament.

A further aspect of the present invention provides a method of dosing a animal with a medicament comprising the steps of: providing a sealed package within which an animal foodstuff and a predetermined dose of the medicament are contained; opening the package; and feeding the animal with the feed mixture.

In order that the present invention may be more readily understood, embodiments of the invention will now be described, by way of example.

A preferred embodiment of the present invention comprises a prepared foodstuff suitable for consumption by a particular type of animal such as, for example, a cat or a dog. A medicament such as a prophylactic for the prevention of parasitic infestation is also provided and a predetermined dose of the medicament is thoroughly admixed with the foodstuff. The resultant feed mixture of foodstuff and medicament is then packaged such that the package contains a measure of the nourishing foodstuff and a predetermined dose of the medicament. The foodstuff remains palatable to the animal even though the medicament has been admixed thereto, the volume of medicament compared to the volume of foodstuff being low enough that the medicament is effectively unnoticed by the animal.

The foodstuff may be ay kind, or mixture of, wet, moist, or dry animal foodstuff.

Preferably, the package is in the form of a carton, a tin or a vacuum sealed package although other packaging alternatives are possible. In one embodiment of the invention, it is envisaged that the foodstuff and the medicament are held in the same package but in separate compartments so as to minimise any possible reaction between the foodstuff and the medicament. When the feed mixture is to be fed to the animal, the dividing barrier between the medicament and the foodstuff can be broken and the foodstuff mixed with the medicament within the package which remains unopened. Preferably, the dividing barrier can be ruptured or burst by the application of deliberate and sufficient pressure to the barrier thereby allowing mixing of the foodstuff and the medicament within the unopened package.

The packaged foodstuff incorporating the predetermined dose of medicament solves the problems associated with regular dosing of animals, particularly domestic pets. All the animal keeper has to do is to purchase an appropriate package containing a foodstuff and the required dose to be regularly administered and feed the feed mixture to the animal. The feed mixture provides a complete and sufficient repast for the animal as well as the predetermined dose of medicament.

The packages are advatageously available in different sizes which can be administered singly or in multiples or combinations in order to achieve the correct therapeutic dose for a given animal, usually depending on its type and size. For example, the packages may be available as large tins, small tins and cartons, the sizes of the packages being such that one large tin is equivalent in size to two small tins or four cartons. A single carton would be sufficient to feed a cat, a small tin to feed a Corgie dog, a large tin to feed a Collie dog, a large tin and a small tin to feed a Labrador dog and two large tins to feed a Great Dane dog. It is envisaged that packages for dogs would include different foodstuffs than those for cats.

Because of the unitary nature of the predetermined dose of the medicament within each package, dosages can be varied by feeding a given animal packages in one or more sizes which can be administered singly, in multiples or in combinations thereof in order to achieve the correct therapeutic dose for the given animal.

It should be appreciated that feed mixtures embodying the present invention provide a measured dose of medicine(s) for the cure and prophylaxis of parasite infestation in domestic pets and other animals, administered as a complete and sufficient repast without the need for adding and mixing any other elements.

The features disclosed in the foregoing description, in the following claims and/or in the accompanying drawings may both separately and in any combination thereof, be material for realising the invention in diverse forms thereof.

## Claims

1. A feed mixture comprising a sealed package containing an animal foodstuff and a predetermined dose of a medicament.

2. A feed mixture according to Claim 1, wherein the foodstuff and the predetermined dose of the medicament are pre-mixed together in the package.

3. A feed mixture according to Claim 1, wherein the package is provided with a dividing barrier to separate the foodstuff and the predetermined dose of the medicament into separate compartments in the package.

4. A feed mixture according to Claim 3, wherein the barrier is breakable within the package without opening the package to allow mixing of the foodstuff and the predetermined dose of the medicament within the package.

5. A feed mixture according to any preceding claim, wherein the medicament comprises one or more pharmaceuticals, drugs, medicines, antibiotics or vaccines in exact quantities, the feed mixture being intended to feed and simultaneously to deliver an accurate medicinal dose for a specific type or size of animal.

6. A feed mixture according to any preceding claim, wherein the medicament is a prophylactic for the prevention of infestation of the specific animal with any or all kinds of worm or other intestinal or other parasite, internal or external.

7. A feed mixture according to any preceding claim, wherein the animal to be fed the feed mixture is a domestic animal.

8. A feed mixture according to any preceding claim, wherein the animal to be fed the feed mixture is a non-domestic animal.

9. A feed mixture according to any preceding claim, wherein the predetermined dose of the medicament comprises a unitary dose, the number of unitary doses required to dose a particular animal being dependent on the type and size of the animal.

10. A method of dosing an animal with a medicament comprising the steps of: providing a sealed package within which a animal foodstuff and a predetermined dose of the medicament are contained; opening the package; and feeding the animal with the feed mixture.

11. A method according to Claim 10, wherein the animal foodstuff and the predetermined dose of the medicament are pre-mixed together within the package.

12. A method according to Claim 10, wherein the animal foodstuff and the predetermined dose of the medicament are separated from one another within the package.

13. A method according to Claim 12, wherein a dividing barrier is provided within the package to separate the animal foodstuff from the predetermined dose of the medicament, the barrier being breakable without the package being opened.

14. A method according to Claim 11 or 12, wherein the animal foodstuff and the predetermined dose of the medicament are mixed together within the package after the barrier is broken and before the package is opened.
